# EUROPEAN PATENT SPECIFICATION

(11) **EP 2 624 667 B1**
(45) Date of publication and mention of the grant of the patent: **15.03.2017**
(21) Application number: 10788355.5
(22) Date of filing: 28.09.2010
(51) Int. Cl.: H05G 1/34, H05G 1/46, H05G 1/10

(54) **METHOD FOR THE AUTOMATIC CONTROL OF MAXIMUM POWER FOR X-RAY APPARATUSES, AND DEVICE REQUIRED FOR SAME**
VERFAHREN ZUR AUTOMATISCHEN STEUERUNG DER HÖCHSTLEISTUNG FÜR RÖNTGENGERÄTE UND DAFÜR ERFORDERLICHE VORRICHTUNG
PROCÉDÉ DE RÉGULATION AUTOMATIQUE DE PUISSANCE MAXIMALE POUR UN ÉQUIPEMENT À RAYONS X ET DISPOSITIF NÉCESSAIRE À CETTE FIN

(43) Date of publication of application: 07.08.2013
(73) Proprietor: Sociedad Española De Electromedicina Y Calidad, S. A., 28110 Algete (ES)
(72) Inventor: DÍAZ CARMENA, Angel, E-28110 Algete (ES); GÓMEZ RODRIGUEZ, Aníbal, E-28110 Algete (ES); DÍAZ CARMENA, Francisco, E-28110 Algete (ES)
(74) Representative: Monzón de la Flor, Luis Miguel
(86) International application number: PCT/ES2010/070624
(87) International publication number: WO 2012/042067

(56) References cited:
- EP-A2- 0 351 508
- JP-A- 61 126 800
- US-A- 4 540 930
- US-A- 4 789 998
- US-A- 5 329 568
- US-A1- 2005 231 178

## Description

### OBJECT OF THE INVENTION

The object of the invention, as described by its title, is a method for automatic maximum power regulation for an x-ray unit, whether battery-powered or connected to the power grid, and the device that carries out this method.

The present invention is characterised by the special features of the method stages, which allow supplying the maximum power to the x-ray unit at all times, allowing to obtain x-ray images with optimum quality for the unit.

### BACKGROUND OF THE INVENTION

Portable x-ray units or movable x-ray units connected to the power grid suffer from a common drawback: the voltage drop in the power line at the time of discharge, which is unknown and even unpredictable, and therefore cannot be compensated for. This voltage drop at the time of discharge completely distorts the x-ray image (because the necessary kVp are not reached), resulting in an image that does not allow analysing the patient's internal condition.

This is particularly relevant in home medical assistance, where a portable x-ray unit is used to take an x-ray image of a person who, for whatever reason, cannot be taken to a hospital. It is also relevant in farms when veterinaries wish to take an x-ray of an animal, or when a power cable is deployed to the portable x-ray unit.

In all of these cases, whether due to the power line itself or to the use of an extension cord, a voltage drop usually occurs at the time of discharge. This distorts the radiographic image so that the part of interest cannot be seen clearly.

The solution hitherto used for these problems is to reduce the unit's power. Thus, for example, 4 kW units are made to work at 1 kW power levels, which reduces the unit's capabilities, with the resulting dissatisfaction of users and buyers of these mobile units.

An identical problem occurs in x-ray units powered by batteries, as the batteries have an internal resistance that increases over time due to battery ageing and oxidation, reducing the maximum power that the batteries can supply. This is, units with aged batteries cannot produce x-ray images of sufficient quality.

Therefore, the object of the present invention is to develop a method for automatic regulation of the maximum power supplied to portable x-ray units, whether these are powered by the power grid or by batteries, so that they can supply the maximum power at the time of discharge, thereby preventing the loss in quality of the x-ray images.

EP 0 351 508 A2 discloses a method for automatically matching an X-ray diagnostic generator with the mains resistance. According to this method, the actual values of the grid voltage and of the voltage drop are measured and the internal resistance values are calculated. For the desired X-ray tube voltage and mAs product, the shortest recording time at the highest allowable current is calculated according to stored data; then the necessary grid voltage for obtaining the desired condition is calculated and compared with the actual available value. If the available value is not sufficient, then the X-ray tube current is reduced and a longer recording time is calculated.

### DESCRIPTION OF THE INVENTION

The object of the invention is a method for automatic regulation of the maximum power delivered to an x-ray unit, with the aim of having maximum power delivered at all times.

The process for obtaining x-ray images has two basic values or parameters that allow obtaining images of different type. On one hand, the peak value of the voltage applied, expressed as kVp, and on the other the product of the mA supplied and the exposure time, also known as mAs.

The peak kilovolts value gives the penetration of the radiation. An image of a rib is not the same as that of an internal organ such as a lung; to see the organ through the bone it is necessary to raise the value of kVp so that it can pass through the bone easily.

The product of the milliamperes and exposure time (mAs) make the image clearer or darker.

However, if the value of the kV applied is not appropriate, because the grid or the batteries cannot supply this voltage value, the resulting images have insufficient quality. On another hand, it is known that the power supply circuit consists of a power supply (of the grid or line or of the batteries) connected in series to a resistance, which in the case of x-ray units powered by batteries is the internal resistance of the batteries and in the case of x-ray units powered by the grid or a supply line is the line resistance. The terminals of this circuit are connected to a resistance, as it is known that the maximum power is obtained at the connection ends of the resistance when the external resistance connected is equal to the internal resistance of the batteries or the line resistance, and therefore when the voltage at the terminals of the external resistance is half of the power supply voltage.

It is known that for an x-ray unit operating with universal voltage (90VAC to 264VAC) the minimum power supply voltage for operating the x-ray unit is 90 volts, so that the power supply voltage should never be less than this value, even if it is not the same as the maximum power voltage. For example, for a unit connected to 120V the maximum power is obtained at 60V, but the unit cannot operate under 90V. Units connected to batteries do not have this limitation, as they are designed specifically for this purpose and can always operate at optimum power.

Therefore, the object of the operating method for the x-ray unit is to supply the maximum power at all times, for which the supply or input voltage at the x-ray tube must be half the power supply voltage ((Vbat)/2 or (Vline)/2), referred to as the reference voltage (Vref); in case of a deviation from the reference voltage, the method modifies the current supplied, changing the exposure time so that the maximum power is delivered at all times. In short, it is based on a method that regulates the tube current as a function of the supply voltage (of the batteries or the grid).

The invention is defined in the appended claims.

### DESCRIPTION OF THE DRAWINGS

To complete the description made below and to aid a better understanding of its characteristics, the present descriptive memory is accompanied by a set of drawings, the figures of which represent the most significant details of the invention for purposes of illustration only and in a non-limiting sense.
Figure 1 shows the simplified wiring scheme in the maximum power supply conditions.
Figure 2 shows a representation of the microprocessor in charge of calculating the reference voltage.
Figure 3 shows the representation of the stages for calculating the reference voltage.
Figure 4 shows a representation of the power control process.
Figure 5 shows a representation of the device needed to carry out this method.

### PREFERRED EMBODIMENT OF THE INVENTION

In view of the figures, a preferred embodiment of the proposed invention is described below.

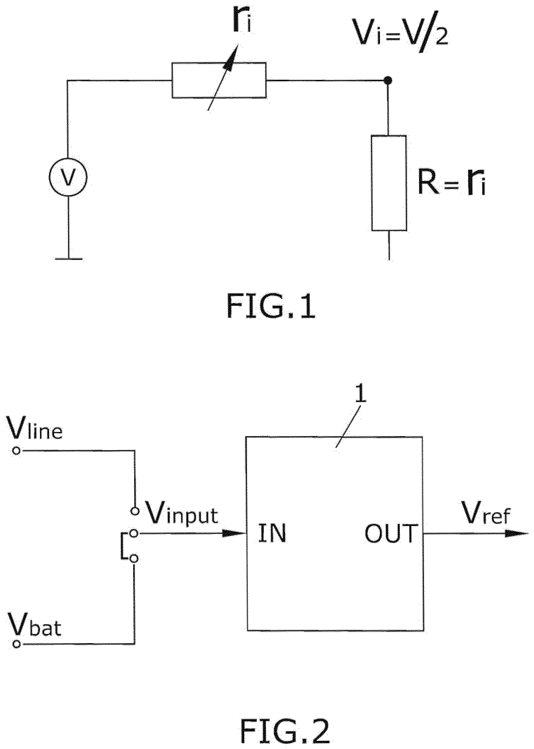

Figure 1 shows the simplified power supply circuit for an x-ray unit, where (V) is the power supply voltage (voltage provided by the batteries (V_{bat}) or the power grid (Vₗᵢₙₑ)), (ri) is the resistance in series with the power supply voltage, which is generally variable and in the case of battery power corresponds to the internal resistance of the batteries and in the case of grid power supply corresponds to the line resistance.

Connected in parallel to this (V) and (rᵢ) series assembly is a resistance (R), which corresponds to the resistance of the x-ray unit. The maximum power is delivered when R is equal to ri, so that the unit input voltage (Vi) is equal to half of the voltage of the power supply ((V_{bat}/2) or (Vₗᵢₙₑ/2).

Figure 2 shows a microprocessor (1) in charge of calculating the reference voltage (Vre
f) for obtaining the maximum power. This microprocessor receives as signals either the line voltage (Vₗᵢₙₑ) or the battery voltage (V_{bat}).

As indicated above, the unit cannot operate at voltages under 90 Volts.

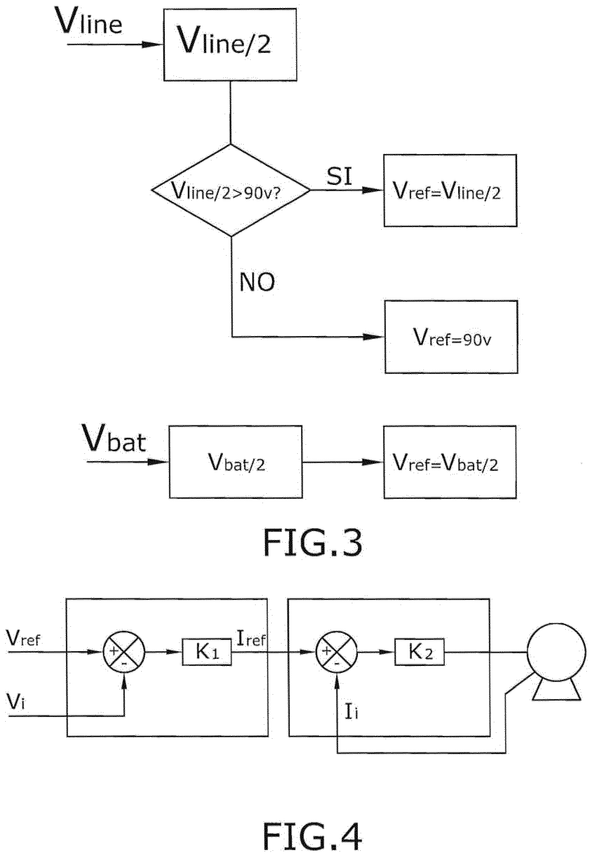

Figure 3 shows the method used to obtain (Vref), which will be equal to (Vline/2), for supply by the grid, or (Vbat/2) for supply by a battery pack, provided (Vline/2) is greater than 90 Volts; otherwise, Vref will be 90 Volts.

Figure 4 shows the power control process, which has a first loop (2) for regulating the
power (2) followed by a second loop (3) for controlling the filament current (Ifilament).

The first regulation loop (2) compares the reference voltage (Vref) and the input voltage (Vi) to obtain a reference value for the current (I ref).

The second regulation loop (3) compares the reference current (Iref) and the input current (Iᵢ), obtaining the filament current (Ifilament), this is, the current that must be supplied to the x-ray tube.

In short, the aim is to have the x-ray unit deliver the maximum power at all times. As this occurs when the input voltage is half of the line voltage (Vline) or the battery voltage (Vbat), and as when a discharge takes place the optimum voltage required of the grid or batteries tends to be maintained, the reference voltage is maintained, changing the filament current (Ifilament) and changing the exposure time.

This is a dynamic and continuous process that corrects at all times the value of the tube filament current, so that if there is a deviation from the reference voltage the filament current to the x-ray tube is changed.

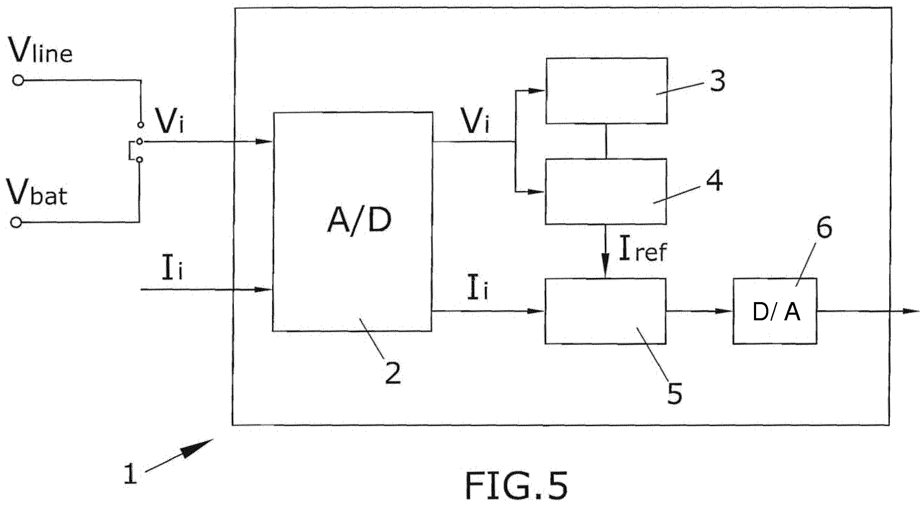

Figure 5 shows a schematic representation of the parts or characteristics of the device used to carry out the method described above. This device comprises a microprocessor (1) having:
- An A/D converter (2) for the input voltage Vi and input current
- A part (3) dedicated to calculating and establishing a reference voltage (Vref)
- A part (4) dedicated to regulating the power, comparing the reference voltage (Vref) and the input voltage (Vi)
- A part (5) in charge of regulating the filament current (Ifilament) that compares the value of the input current (Ii) to the value of the reference current (Iref)
- A D/A converter (6) in charge of supplying the filament current (Ifilament) to the x-ray tube.

The essence of this invention is not affected by variations in the materials, shape, size and arrangement of its component elements, described in a non-limiting manner that will allow its reproduction by an expert

## Claims

1. Method for automatic maximum power regulation in x-ray units comprising an X-ray tube, the method comprises the following stages:
- First, calculating a reference voltage (Vref), which if the x-ray unit is powered by the grid will be half of the line voltage (V line), provided this value is greater than 90 volts, and otherwise the reference voltage will be 90 volts; and if it is battery powered the reference voltage will be half of the voltage supplied by the batteries (V bat);
- Then, comparing the reference voltage (Vref) and an input voltage (Vi) supplied to the x-ray unit, to obtain a reference value for a reference current to supply (Iref);
- then, regulating a filament current (Ifil), which is the current that must be supplied to the X-ray tube, by comparing the reference value obtained for the reference current (Iref) to an input current (Ii) to obtain the filament current (Ifil), which is supplied to the x-ray tube, for maintaining the input voltage (Vi) at the reference voltage (Vref).

2. Device for carrying out the method claimed above, wherein said device comprises a microprocessor (1) having:
- An A/D converter (2) for the input voltage (Vi) and input current (li)
- A part (3) dedicated to calculating and establishing a reference voltage (Vref) which will be half of the line voltage (V line), provided this value is greater than 90 volts, and otherwise the reference voltage will be 90 volts; and if it is battery powered the reference voltage will be half of the voltage supplied by the batteries (V bat).
- A part (4) dedicated to regulating the power, comparing the reference voltage (Vref) and the input voltage (Vi).
- A part (5) in charge of regulating the filament current (Ifilament) that compares the value of the input current (li) to the value of the reference current (Iref).
- A D/A converter (6) in charge of supplying the filament current (Ifilament) to the x- ray tube.

## Patentansprüche

1. Verfahren zur automatischen Steuerung der Höchstleistung in Röntgengeräten, die eine Röntgenröhre umfassen, wobei das Verfahren folgende Stufen umfasst:
- Zunächst die Berechnung einer Referenzspannung (Vref), die bei Netzspeisung des Röntgengeräts halb so groß ist wie die Netzspannung (V line), sofern dieser Wert größer als 90 Volt ist, und andernfalls die Referenzspannung 90 Volt beträgt; und wobei bei Batteriebetrieb die Referenzspannung halb so groß ist wie die von den Batterien gelieferte Spannung (V bat);
- Danach den Vergleich der Referenzspannung (Vref) mit einer in das Röntgengerät eingespeisten Eingangsspannung (Vi), um einen Referenzwert für einen einzuspeisenden Referenzstrom (Iref) zu erhalten;
- Anschließend die Steuerung eines Heizfadenstroms (Ifil), der der in die Röntgenröhre einzuspeisende Strom ist, durch Vergleich des für den Referenzstrom (Iref) erhaltenen Referenzwertes mit einem Eingangsstrom (li), um den Heizfadenstrom (Ifil) zu erhalten, der in die Röntgenröhre eingespeist wird, um die Eingangsspannung (Vi) auf dem Niveau der Referenzspannung (Vref) zu halten.

2. Vorrichtung zur Ausführung des Verfahrens nach dem vorhergehenden Anspruch, wobei die besagte Vorrichtung einen Mikroprozessor (1) umfasst, der
- einen AD-Wandler (2) für die Eingangsspannung (Vi) und den Eingangsstrom (Ii);
- eine Einheit (3) zur Berechnung und Festlegung einer Referenzspannung (Vref), die halb so groß ist wie die Netzspannung (V line), sofern dieser Wert größer als 90 Volt ist, und andernfalls die Referenzspannung 90 Volt beträgt; und bei Batteriebetrieb die Referenzspannung halb so groß ist wie die von den Batterien gelieferte Spannung (V bat);
- eine Einheit (4) zur Steuerung der Leistung durch Vergleich der Referenzspannung (Vref) mit der Eingangsspannung (Vi);
- eine Einheit (5) zur Steuerung des Heizfadenstroms (Ifilament), die den Wert des Eingangsstroms (Ii) mit dem Wert des Referenzstroms (Iref) vergleicht;
- einen DA-Wandler (6) zur Versorgung der Röntgenröhre mit dem Heizfadenstrom (Ifilament)
aufweist.

## Revendications

1. Méthode de régulation automatique de puissance maximale pour des équipements à rayons X comprenant un tube à rayons X, laquelle méthode comprend les étapes suivantes :
- dans un premier temps, calcul d'une tension de référence (Vref), qui sera égale à la moitié de la tension du secteur (V line) si l'équipement à rayons X est alimenté par le secteur, à condition que cette valeur soit supérieure à 90 volts, dans le cas contraire la tension de référence sera de 90 volts ; et si l'équipement est alimenté par batteries, la tension de référence sera égale à la moitié de la tension fournie par les batteries (V ^{bat}) ;
- dans un deuxième temps, comparaison de la tension de référence (Vref) avec la tension d'entrée (Vi) fournie à l'équipement à rayons X, afin d'obtenir une valeur de référence pour un courant de référence à fournir (Iref). ;
- finalement, régulation du courant d'un filament (Ifil), lequel est défini comme le courant à fournir au tube à rayons X, en comparant la valeur de référence obtenue pour le courant de référence (Iref) avec un courant d'entrée (Ii) afin d'obtenir le courant de filament (Ifil), lequel est fourni au tube à rayons X, pour maintenir la tension d'entrée (Vi) à la tension de référence (Vref).

2. Dispositif destiné à l'implémentation de la méthode revendiquée ci-dessus, **caractérisé en ce que** ledit dispositif comprend un microprocesseur (1) qui possède :
- Un convertisseur A/N (2) pour la tension d'entrée (Vi) et le courant d'entrée (Ii)
- Une partie (3) destinée à calculer et à établir une tension de référence (Vref) qui sera égale à la moitié de la tension de ligne (V line), à condition que cette valeur soit supérieure à 90 volts, dans le cas contraire la tension de référence sera de 90 volts ; et s'il est alimenté par batteries, la tension de référence sera égale à la moitié de la tension fournie par les batteries (V bat).
- Une partie (4) prévue pour la régulation de la puissance, afin de comparer la tension de référence (Vref) avec la tension d'entrée (Vi).
- Une partie (5) chargée de la régulation du courant de filament (Ifilament) qui compare la valeur du courant d'entrée (Ii) avec la valeur du courant de référence (Iref).
- Un convertisseur N/A (6) chargé de fournir le courant de filament (Ifilament) au tube à rayons X.
